**Europäisches Patentamt**

**European Patent Office**

**Office européen des brevets**

(11) Numéro de publication: **0 001 197**
**B1**

## (12) FASCICULE DE BREVET EUROPEEN

(45) Date de publication du fascicule de brevet: **04.11.81**

(21) Numéro de dépôt: **78400090.3**

(22) Date de dépôt: **05.09.78**

(51) Int. Cl.³: **C 07 G 17/00,**
**G 01 N 33/48, A 61 K 39/00**

(54) Produits de couplage de la cytidine-diphosphocholine et de composés aminés, procédé d'obtention et application pharmaceutique et analytique.

(30) Priorité: **07.09.77 FR 7727151**

(43) Date de publication de la demande:
**21.03.79 Bulletin 79/6**

(45) Mention de la délivrance du brevet:
**04.11.81 Bulletin 81/44**

(84) Etats Contractants Désignés:
**BE CH DE GB NL**

(56) Documents cités:
**CHEMICAL ABSTRACTS, vol. 61, 13749h (1964)**

**C.R. ACAD. SC. Paris, Série D, vol. 281,
203—206 (1975) "conclusions"**

**THE JOURNAL OF IMMUNOLOGY, vol. 118, n° 6,
1987—94 (1977)**

**NATURE, vol. 247, 55—57 (1974)**

(73) Titulaire: **Etablissement public dit: INSTITUT
NATIONAL DE LA RECHERCHE AGRONOMIQUE
149, rue de Grenelle
F-75341 Paris Cedex 07 (FR)**

(72) Inventeur: **Pery, Pierre
15, Impasse du Moulin à vent-Le Prioré
F-78450 Villepreux (FR)**
Inventeur: **Luffau, Gérard
Le Petit Bontemps-Bâtiment 8 Escalier 23
F-78370 Plaisir (FR)**
Inventeur: **Poulain, Joelle épouse Charley
4 Rue du Petit Bontemps Residence Villiers
F-78370 Plaisir (FR)**
Inventeur: **Petit, Agnès
Station de Recherche de Virologie
F-78850 Thiverval Grignon (FR)**

(74) Mandataire: **Harlé, Robert et al,
c/o Cabinet Harlé & Lechopiez 21, rue de la
Rochefoucauld
F-75009 Paris (FR)**

Courier Press, Leamington Spa, England.

# 0 001 197

Produits de couplage de la cytidine-diphosphocholine et de composés aminés, procédé d'obtention et application pharmaceutique et analytique.

La présente invention concerne des produits de couplage de la cytidine-diphosphocholine et de composés aminés; elle a également pour objet un procédé pour l'obtention de ces produits et leurs applications, notamment leurs applications pharmaceutiques, en particulier comme vaccins.

La phosphorylcholine est un composé simple, qui est lié dans la nature à des lipides, des polyosides et des protéines. Des essais ont en effet montré que les extraits des différents stades de développement de plusieurs nématodes: *Nippostrongylus brasiliensis* (parasite du rat), *Haemonchus contortus* (parasite du mouton), des nématodes non déterminés de l'appareil respiratoire du porc et *Heligmosomoïdes polygyrus* (parasite de la souris) contenaient des composés macromoléculaires portant de la phosphorylcholine; ces composés peuvent être mis en évidence grâce à des myélomes de souris possédant une activité anticorps antiphosphorylcholine; à cet effet, on peut se référer à l'article de PERY et al. dans European Journal of Immunology vol. 4, 1974, pp. 637—639.

L'étude immunochimique du composé macromoléculaire portant de la phosphorylcholine, obtenu à partir de *Nippostrongylus brasiliensis* et purifié, a révélé un déterminant antigénique commun à tous les extraits testés [PERY et al. 1975 b. Ann. Immunol. (Institut Pasteur) *126* C 335].

Par ailleurs, des essais ont montré que l'injection par voie intradermique de 10 $\mu$g du composé purifié ci-dessus à des rats, huit jeurs avant une infestation, se traduit par une modification du transit des larves dans les poumons et par une diminution du nombre des parasites s'installant dans l'intestin avant l'autostérilisation [PERY et al. 1975 a C.R. Acad. Sc. Paris *281* série D 203 206].

On a déjà proposé de préparer des produits conjugés entre des protéines et des composés porteurs de groupes phosphorylcholine. Ainsi, J. Schroer et J.M. Davie dans "The Journal of Immunology" Vol. 118, n° 6 1987—94 (1977) ont décrit le couplage de phosphorylcholine avec l'hémocyanine et identifié les fractions d'antigène formées lors de l'injection de ces conjugués à des cobayes. De même, W. Lee, H. Cosenza et H. Köhler ont décrit dans "Nature", Vol. 247, 4 janvier 1974, le couplage de p-diazophénylphosphorylcholine avec les résidus tyrosine de protéines, telles que l'hémocyanine ou les flagelles de Salmonelles. Les mêmes auteurs ont étudié la réponse immunitiaire des souris à l'injection de ces conjugés.

Cependant, les synthèses des produits conjugués de la technique antérieure, porteurs de groupes phosphorylcholine, sont compliquées et les composés de départ sont peu accessibles. De plus le couplage se fait par l'intermédiaire des résidus tyrosine des protéines ce qui limite donc la généralité de la technique de couplage et la nombre de groupes phosphorylcholine qui peuvent être fixés sur une mole de protéine.

L'invention permet d'éliminer ces inconvénients des procédés et produits de la technique antérieure.

On a maintenant trouvé qu'il est possible d'obtenir, par synthèse, des produits à base de phosphorylcholine qui constituent des antigènes artificiels chimiquement définis.

Les produits de l'invention sont des produits de couplage de cytidine-diphosphocholine; et de composés aminés portant des residues lysine, la liaison de couplage se faisant, après oxydation de la cytidine-diphosphocholine, entre les groupes ribose oxydés de cette dernière et les residues lysine desdits composés aminés ils sont utilies comme vaccins anti-parasitaires. Une autre application des composés de l'invention se situe dans le domaine du dépistage, de la purification et du dosage des composés biologiques réagissant avec la phosphorylcholine, c'est-à-dire de la protéine C réactive et des anticorps anti-phosphorylcholine.

Les produits de l'invention sont obtenus par couplage entre la cytidine-diphosphocholine et lesdits composés aminés; le procédé de l'invention pour l'obtention de ces composés de couplage consiste à faire réagir la cytidine-diphosphocholine avec des composés aminés après oxydation, selon la technique mise au point par ERLANGER et BEISER pour la fixation de ribonucléotides sur les protéines [1964, Proc. Nat. Acad. Sci. USA, *52*, 68—74].

Sous une forme plus détaillée, le procédé selon l'invention comprend les étapes suivantes qui consitent:

1) à oxyder de la cytidine-diphosphocholine;

2) à fixer le dialdéhyde, obtenu par l'oxydation précédente du ribose de la cytidine, sur les groupements $NH_2$ du composé aminé en solution aqueuse alcaline;

3) à stabiliser le couplage ainsi réalisé par réduction;

4) à récupérer le produit macromoléculaire ainsi obtenu sous forme d'une solution aqueuse, celle-ci pouvant être lyophilisée pour fournir une poudre apte à la conservation.

Les composés aminés qui conviennent aux fins de la présente invention sont des composés portant des résidus lysine. Par composés portant des résidus lysine, on entend les composés qui possèdent naturellement des résidus lysine et les composés obtenus per Fixetion de Résidus lysine sur des composés sur lesquels on peut fixer des résidus lysine; ce sont par exemple des protéines, des peptides de synthèse, des polysaccharides modifiès par des résidus lysine. A titre d'exemples de composés aminés appropriés, on peut citer les albumines des différentes espèces animales, notamment la sérum albumine humaine, l'ovalbumine, la gélatine, les lectines comme la concanavaline A,

2

l'hémocyanine, la limule, les corps bactériens, les flagelles de salmonelles, les bactériophages, les virus, les enzymes, les polymères de la lysine ayant un degré de polymérisation supérieur à 2, les polymères synthétiques d'acides aminés possédant plusieurs résidus de lysine. Parmi les composés ne portant pas de résidus lysine, mais sur lesquels on peut aisément fixer de la lysine, on peut citer les polysaccharides tels que les dextranes, levanes et les polymères de sucrose et d'épichlorhydrine, tels que par exemple le produit connu sous la dénomination commercialle "FICOLL" (fabriqué par Pharmacia Fine Chemicals, Uppsala, Suède) et d'autres composés analogues. Lorsqu'on met en oeuvre un composé ne comportant pas de résidus lysine mais sur lequel on peut fixer aisément de la lysine, tel qu'un polysaccharide par exemple, on procède d'abord à la fixation de la lysine sur ce composé pour obtenir un polysaccharide modifié par de la lysine et on réalise ensuite la réaction de couplage selon l'invention entre ce polysaccharide modifié et la cytidine-diphosphocholine.

L'oxydation du ribose de la cytidine-dipphocholine peut être avantageusement réalisée avec du periodate de sodium. Il convient d'utiliser un excès d'agent oxydant par rapport à la cytidine-diphosphocholine à oxyder; l'excès d'agent oxydant peut ensuite être aisément éliminé, par exemple par décomposition.

La solution aqueuse alcaline du composé aminé mise en oeuvre dans l'étape 2 du procédé de l'invention a un pH compris entre environ 9 et 9,5; le pH de la solution a aqueuse est avantageusement ajusté à la valeur ci-dessus à l'aide de carbonate de sodium 5%.

Le couplage ainsi réalisé est ensuite stabilisé par réduction; à titre d'agent réducteur convenable, on peut citer le borohydrure de sodium ou tout autre composé analogue.

Le produit macromoléculaire formé est ensuite séparé des réactifs, par exemple par chromatographie sur gel, et est enfin avantageusement lyophilisé.

Le nombre de moles de cytidine-diphosphocholine par mole de composé aminé dépend du composé aminé considéré; en général, ce nombre de moles est inférieur ou égal au nombre de groupes $NH_2$ dans le composé aminé. Dans le cas de la sérum albumine humaine, le rapport des restes de phosphorylcholine au nombre de moles de sérum albumine humaine est avantageusement compris entre 1 et 30.

Les produits de l'invention sont des produits de couplage de cytidine-diphosphocholine et de composés aminés choisis parmi les composés portant des résidus lysine et les composés obtenus par Fixetion de résidus lysine sur des composés sur lesquels on peut fixer de la lysine, la liaison de couplage se faisant, après oxydation de la cytidine-diphosphocholine, entre les groupes ribose oxydés de cette dernière et les résidus lysine desdits composés aminés.

L'invention utilise comme produit de départ la cytidine-diphosphocholine qui est une substance disponible dans le commerce et peu coûteuse. Contrairement à l'art antérieur, le procédé de l'invention implique un petit nombre d'étapes. Il est aisément transposable à l'échelle industrielle. Ne nécessitant pas la mise en oeuvre de solvant votatil, il évite des étapes supplémentaires et coûteuses d'évaporation ou d'élimination ultérieure. de ce solvant, par exemple l'acétonitrile ou la pyridine.

La cytidinediphosphocholine est par ailleurs un composé de structure connue, qui est un intermédiaire dans la synthèse des lécithines et des sphingomyélines. Les produits conjugués, préparés selon l'invention à partir de cytidine- diphosphocholine, sont dégradables in vivo; ils n'entraînent donc pas de réaction secondaire car ils sont utilisables dans le métabolisme normal des cellules. Au contraire, les produits conjugués de l'art antérieur, qui sont obtenus par exemple à partir de p-diazophénylphosphorylcholine, peuvent entraîner des réactions indésirables dans un organisme vivant.

Les produits de l'invention se caractérisent par le fait que le couplage se fait par l'intermédiaire des résidus lysine du composé aminé, alors que dans l'art antérieur, la fixation se fait par les groupes tyrosine. Les groupes lysine sont en général plus nombreux sur les composés aminés que les groupes tyrosine, ce qui permet selon l'invention de fixer davantage de groupes phosphorylcholine sur un composé aminé déterminé.

Les produits de couplage selon l'invention sont de plus caractérisés par

—1) leur masse molaire qui est voisine de celle du composé support du fait que la masse molaire de la cytidine-diphosphocholine est très faible vis-à-vis de celles de composés supports;

—2) leur mobilité électrophorétique qui est plus rapide que celle du composé à pH 8,6. En effet, on note, grâce à des immunoélectrophorèses révélant les antigènes de la molécule support, une augmentation de la vitesse de migration en fonction du degré de substitution du composé;

—3) leur spectre d'absorption en lumière ultraviolette; les spectres U.V. des protéines possèdent deux longueurs d'ondes caractéristiques qui permettent de déterminer le degré de couplage: 280nm (absorption des groupements tyrosine et phénylalanine des protéines); 260nm (absorption de la cytosine de la cytidine diphosphocholine); et

—4) leur teneur moyenne en résidus de phosphorylcholine par molécule de composé support.

La caractéristique 3) ci-dessus permet une mesure exacte du degré de couplage obtenu, par simple comparaison des spectres U.V. du composé aminé d'une part et du produit conjugué, d'autre part.

On notera également que le procédé de l'invention est applicable de façon très générale, non seulement à des protéines, mais aussi à des composés aminés autres que des protéines, la seule condition étant que l'on puisse fixer de la lysine sur ces composés. L'invention permet donc une grande variété de couplage.

Comme on l'a indiqué précédemment, les produits de couplage de l'invention sont utiles comme vaccins antiparasitaires.

L'étude de la physiopathologie des animaux parasités et de l'acquisition d'une résistance à la réinfestation (soit à la suite d'une primo-infestation, soit à la suite d'une vaccination) est difficile, lorsque l'expérimentation est pratiquée sur des aminaux domestiques. Cependant, on a trouvé que le cycle évolutif de certains parasites, tels que par exemple *Nippostrongylus brasiliensis*, nématode parasite de l'intestin du rat, présente de grandes analogies avec celui de certains des parasites des animaux domestiques. Les produits de couplage selon l'invention ont donc été testés sur le rat.

Le rat est capable, au bout d'une diazaine de jours, de stopper la ponte des oeufs des parasites femelles adultes *Nippostrongylus brasiliensis*, puis de se débarrasser de sa population de vers intestinaux. Ce phénomène d'autostérilisation est considéré comme la première manifestation de l'immunité suscitée chez l'hôte par le parasite. Le mécanisme de l'autostérilisation semble complexe et nécessite vraisemblablement l'action conjuguée des anticorps et de cellules lymphoïdes immunes.

Une seconde infestation des rats après l'autostérilisation montre que ces derniers sont devenus résistants au parasite.

Cette résistance peut étre obtenue à la suite d'une infestation par une très faible quantité de larves infestantes [LUFFAU et al. 1975 Ann. Immunol. (Institut Pasteur) *126* C 354].

On a trouvé que l'administration de produits de couplage selon l'invention à des rats douze jours avant une infestation par le parasite *Nippostrongylus brasiliensis* entraîne une diminution importante des oeufs émis journellement pendant la période de reproduction des parasites et une diminution aussi importante des vers adultes contenus dans l'intestin au cours de la période prédédant l'autostérilisation.

Cette protection est spécifique de la phosphorylcholine, puisque les rats ayant reçu les mêmes doses de sérum albumine non substituée sont aussi sensibles que les témoins, et elle est obtenue de façon homogène chez tous les rats traités convenablement.

Les essais relatés ci-après montrent que les produits de couplage selon l'invention présentent un rôle vaccinant contre les parasites appartenant à la classe de ceux comportant des molécules liées à un résidu phosphorylcholine; il s'agit en général de parasites intestinaux ou respiratoires. Les produits de l'invention sont donc susceptibles d'être utilisés comme vaccins antiparasitaires pour les animaux domestiques et l'homme, compte-tenu de l'analogie entre l'évolution de *Nippostrongylus brasiliensis* et certains parasites d'animaux domestiques dont il est question ci-dessus.

Le doses de produit de couplage phosphorylcholine-composés aminés selon l'invention, qui conviennent à titre de vaccins antiparasitaires, varient suivant le parasite et le composé aminé intervenant dans le couplage.

Toutefois, on peut indiquer que ces doses sont généralement faibles et de l'ordre de quelques microgrammes.

A titre d'exemple, on indiquera que le produit de couplage cytidine-diphosphocholine-sérum albumine humaine peut être utilisé à des doses allant de 20 à 80 $\mu$g par rat.

L'invention concerne donc également des vaccins antiparasitaires contenant, à titre de principe actif, une quantité efficace d'un produit de couplage selon l'invention en combinaison avec un véhicule pharmaceutiquement efficace approprié. Parmi les véhicules qui conviennent aux fins de l'invention, on peut citer le sérum physiologique et tous les tampons aqueux.

En outre, le vaccin contenant un produit de couplage selon l'invention peut être administré sous une forme enrobée, telle qu'une forme en gélules ou en liposomes.

Les vaccins selon l'invention peuvent être administrés par voie intradermique, voie orale ou sous forme d'aérosol.

Les produits de couplage cytidine-diphosphocholine-composés aminés de l'invention sont également utiles comme réactifs de dosage dans les techniques de dépistage, de purification et de dosage de composés biologiques réagissant avec la phosphorylcholine, c'est-à-dire la protéine C réactive et les anticorps anti-phosphorylcholine.

L'introduction d'un composé portant de la phosphorylcholine chez un animal ou chez l'homme peut entraîner la synthèse par l'organisme d'anticorps anti-phosphorylcholine qui pourront être mis en évidence dans le sérum des sujets infestés [LEE et al. 1974, Nature, *247*, 55—57].

Ces anticorps anti-phosphorylcholine ne sont pas les seules substances élaborées par l'organisme qui sont capables de réagir avec des composés comportant de la phosphorylcholine. En effet, on a montré depuis longtemps que le sérum d'individus atteints de différentes affections contenait une protéine de mobilité ß, qui a été appelée protéine C réactive, car elle possédait la capacité d'être précipitée par le polysaccharide C du pneumocoque. On a montré que la réaction entre la protéine C réactive et le polysaccharide du pneumocoque était due à la présence sur ce dernier de résidus de phosphorylcholine [VOLANAKIS et KAPLAN 1971, P.S.E.B.M., *136*, 612—614].

Les réactions de la protéine C réactive et des anticorps anti-phosphorylcholine avec des composés portant de la phosphorylcholine sont très voisines et donnent naissance à des précipités, des agglutinats, des floculats selon le type de réaction utilisé.

On a trouvé que les produits de couplage cytidine-diphosphocholine-composés aminés peuvent se combiner avec le substances recherchées dans les liquides biologiques et permettent ainsi de doser ces substances.

Ainsi, les produits de couplage cytidine-diphosphocholine-composés aminés permettent, en utilisant des tampons contenant 0,01% de chlorure de calcium, de dépister les produits recherchés, à savoir la protéine C réactive et les anticorps anti-phosphorylcholine par des réactions d'immunodiffusion double, par le test de l'anneau, le test de floculation.

Les réactions des produits de couplage cytidine-diphosphocholine-composés aminés avec la protéine C réactive peuvent être inhibées par du citrate trisodique à 5%, le produit connu sous la dénomination commerciale "Anaklepton" $5.10^{-3}M$, de la cytidine-5'-diphosphocholine ou de la phosphorylcholine $10^{-3}M$, alors que seuls les deux derniers composés inhibent la réaction des produits de couplage de l'invention avec les anticorps anti-phosphorylcholine, ce qui permet à la fois un dépistage différentiel et une vérification de la spécificité de la réaction.

On peut également obtenir, par les techniques d'insolubilisaiton décrites pour la chromatographie d'affinité, un dérivé utilisable pour capter les composés réagissant avec la phosphorylcholine dans les fluides biologiques. Le citrate ou 1' "Anaklepton" permettent de libérer la protéine C réactive purifiée ainsi en une seule étape, et la phosphorylcholine ajoutée ultérieurement permet de récupérer les anticorps anti-phosphorylcholine.

L'utilisation de protéines substituées en chromatographie d'affinité permet de purifier très rapidement de grandes quantités de protéine C réactive et d'anticorps anti-phosphorylcholine qui pourraient être lysophilisées et servir d'étalon de mesure pour le dosage quantitatif décrit ci-après.

Par des techniques courantes d'immunologie, on peut doser la protéine C réactive et les anticorps anti-phosphorylcholine avec les produits de couplage selon l'invention. On peut notamment utiliser les niques ci-après:

— la turbidimétrie, ou néphélométrie, qui consiste en la mesure du trouble apparaissant au cours de la réaction du produit recherché;

— l'immunodiffusion radiale simple en utilisant le système inverse décrit par VAERMAN et al [1969, Immunochemistry 6, 287—293] permet, en intégrant le produit de couplage selon l'invention dans un support approprié (gélose, agarose, papier, acétate de cellulose), d'obtenir des zones de précipitation dont le diamètre final est directement proportionnel à la quantité de protéine C réactive ou d'anti-phosphorylcholine réagissant;

— la fixation du produit de couplage selon l'invention sur des globules rouges permet de pratiquer des réactions d'hémagglutination passive. Le traitement par le glutaraldéhyde des globules rouges avant ou après la fixation du produit de l'invention permet de les lyophiliser et de les livrer sous une forme facilement utilisable.

La présente invention concerne donc également des réactifs de dosage de composés biologiques réagissant avec la phosphorylcholine; ces réactifs contiennent un produit de couplage selon l'invention fixé sur des globules rouges de mouton éventuellement traités par du glutaraldéhyde, le produit de couplage pouvant éventuellement être activé par du glutaraldéhyde avant ou après fixation. L'invention concerne également un réactif de dosage se présentant sous la forme de gélose contenant un produit de couplage selon l'invention.

L'invention sera décrite maintenant plus en détail dans les exemples illustratifs non limitatifs donnés ci-après.

Exemple 1

Préparation de produits de couplage cytidine-diphosphocholine-protéine.

La technique générale de couplage de la cytidine-diphosphocholine sur les protéines est la suivante:

De la cytidine-diphosphocholine en quantité variable a été oxydé par 2,5 ml de periodate de sodium 0, 1M, pendant 20 minutes à la température ambiante. L'excès de periodate a été décomposé par addition de 0,15 ml d'éthylène glycol molaire qu'on a laissé agir pendant 5 minutes à la température ambiante.

On a dissous 140 mg de la protéine considérée dans 10 ml d'eau distillée amenée à pH 9,5 par du carbonate de sodium 5% et on a fait réagir la cytidine-diphosphocholine oxydée avec cette protéine.

La réaction de fixation du dialdéhyde obtenu par oxydation périodique du ribose de la cytidine sur les groupements aminés de la protéine s'est poursuivie pendant 45 minutes à pH 9—9,5.

La liaison a ensuite été stabilisée par réduction au borohydrure de sodium pendant une nuit (75 mg de borohydrure de sodium sont dissous dans 10 ml d'eau distillée et ajoutés à la préparation).

La stabilité de la liaison a été testée à pH acide: 5 ml d'acide formique molaire ont été ajoutés pendant 1 heure, puis le pH de la solution a été réajusté à 8,5 avec de l'ammoniaque molaire.

Le produit macromoléculaire obtenu a été séparé des réactifs par dessalage, sur une colonne de gel de polyacrylamide sphérique connu sous la dénomination commerciale "Biogel P 30" [fabriqué par BIO—RAD LABORATORIES 2200 Wright Avenue, Richmond Californie], en bicarbonate d'ammonium puis lyophilisé.

Cette technique a été utilisée avec succès pour coupler la cytidine-diphosphocholine aux protéines suivantes: sérum albumine humaine, gélatine, concanavaline, hémocyanine.

# 0 001 197

### Exemple 2

Les produits suivants, dénommés par l'abréviation PC—HSA affectée d'un indice, ont été synthétisés à partir de sérum albumine humaine selon le mode opératoire précédemment décrit dans l'exemple 1.

| Produit de couplage obtenu | Cytidine-diphosphocholine (en g) | Sérum albumine humaine (en g) |
|---|---|---|
| PC HSA$_0$ | 90 | 140 |
| PC HSA$_1$ | 45 | 140 |
| PC HSA$_2$ | 15 | 140 |
| PC HSA$_3$ | 6 | 140 |
| PC HSA$_4$ | 3 | 140 |
| PC HSA$_5$ | 1,5 | 140 |

Les composés obtenus ont été analysés en chromatographie sur colonne de "Sephadex G100"; on a trouvé qu'ils n'étaient pas contaminés par de la cytidine-diphosphocholine libre.

Le degré de substitution, déterminé par mesure de l'absorption de la lumière ultra violette due à la cytidine diphosphocholine d'une part et à l'albumine d'autre part est le suivant:

PC HSA$_0$: 21 moles de cytidine-diphosphocholine par molécule d'albumine
PC HSA$_1$: 15 moles de cytidine-diphosphocholine par molécule d'albumine
PC HSA$_2$: 5 moles de cytidine-diphosphocholine par molécule d'albumine
PC HSA$_2$: 3 moles de cytidine-diphosphocholine par molécule d'albumine
PC HSA$_4$: 2 moles de cytidine-diphosphocholine par molécule d'albumine
PC HSA$_5$: 1 mole de cytidine-diphosphocholine par molécule d'albumine

En utilisant 140 mg de concanavaline A ou d'hémocyanine et 90 mg de cytidine-diphosphocholine, les produits suivants ont été synthétisés: PC concanavaline A (20 résidus de phosphorylcholine par mole de concanavaline A) et PC hémocyanine (70 résidus de phosphorylcholine par mole d'hémocyanine).

### Exemple 3

Utilisation des produits de couplage cytidine-diphosphocoline-sérum albumine humaine à titre d'agent de dosage.

Les réactions des composés préparés selon l'exemple 1 avec des anticorps anti-phosphorylcholine ou anti-sérum albumine ont été pratiquées selon les deux techniques c-après:

— immunodiffusion double en gélose[Ouchterlony O. Progress in Allergy. S. KARGER Basel 1958. 5, 1]

— hémagglutination passive après fixation des composés sur des globules rouges de mouton [S. LIMIEUX et al. Immunochemistry 1974, 11, 261—269]

Les deux techniques ont donné des résultats identiques:

— tous les composés ont réagi de la méme façon avec des anticorps anti-sérum albumine; il en résulte donc que les résidus phosphorylcholine ne gênent pas la reconnaissance des déterminants de la sérumalbumine.

Les composés PC HSA$_0$ et PC HSA$_1$ ont réagi de la même façon avec des anticorps anti-phosphorylcholine ou de la protéine C réactive, alors que le composé PC HSA$_2$ a moins bien réagi, et les composés PC HSA$_{3,4,5}$ n'ont pas réagi, sans doute à cause d'un degré de substitution trop faible pour donner naissance à un réseau révélable par les techniques utilisées.

Le composé PC HSA$_0$, le plus substitué, a été utilisé pour des déterminations sérologiques semi-quantitatives ou quantitatives de la teneur en anticorps anti-phosphorylcholine ou en protéine C réactive qui sont les deux protéines sériques réagissant avec le motif phosphorylcholine.

Des réactids stables convenant aux applications pratiques ont été obtenus:

— par fixation du PC HSA$_0$ sur des globules rouges de mouton traités par le glutaraldéhyde de façon classique;

— par fixation du PC HSA$_0$ activé par le glutaraldéhyde sur des globules rouges de mouton soumis ou non à un traitement ultérieur par le glutaraldéhyde;

— par la confection de plaques de gélose contenant du PC HSA$_0$ pour pratiquer la méthode de Mancini inversée qui consiste à faire migrer les sérums à tester dans ce gel [VAERMAN et al (1969, Immunochemistry 6 287.293)].

6

# 0 001 197

A l'aide du liquide d'ascite d'un myélome murin secrétant une paraprotéine, ayant une activité anti-phosphorylcholine, de sérum de souris normales ou immunisées contre d'autres antigènes, on a vérifié la spécificité des tests d'hémagglutination pratiqués avec les différents types de globules rouges de mouton mentionnés ci-dessus et du test de Mancini, et on a pu détecter des quantités d'anticorps de l'ordre de $9 \times 10^{-7}$ mg/ml, soit environ 1 pg/ml.

Des sérums humains provenant de l'hôpital Béclère de Clamart ont été classés en deux catégories: ceux qui contenaient de la protéine C réactive et ceux qui en étaient dépourvus, et on a vérifié que le carré du diamètre de l'eanneau de précipitation, obtenu par la technique de Mancini, était proportionnel à la concentration de cette protéine.

La discrimination entre les deux catégories de protéines peut être fait aisément car la réaction de mise en évidence et de dosage de la protéine C réactive n'a pas lieu en l'absence d'ions $Ca^{++}$.

Ces tests peuvent donc être utilisés dans la pratique, en procédant par comparaison entre le sérum à tester et un sérum témoin bien caractérisé. Une réaction d'hémagglutination peut être lue après 2 heures et un test de Mancini après 48 heures.

Des produits de couplage obtenus selon le mode opératoire décrit dans l'exemple 1 lont été également obtenus à partir de l'hémocyanine, de la concanavaline A (voir exemple 2), de la limule et de la gélatine.

Ces composés donnent les mêmes résultats sérologiques que PC—HSA$_0$; le choix de la molécule porteuse de phosphorylcholine pour les réactions sérologiques est donc complètement indifférent, seul compte le degré de substitution qui, théoriquement, doit être égal ou supérieur à 2. On a constaté toutefois qu'expérimentalement les composés portant au moins 5 résidus phosphorylcholine ou plus convenaient comme réactifs de dosage dans le cas particulier de la sérum albumine humaine.

## Exemple 4

Préparation de produits de couplage de la cytidine diphosphocholine avec des composés sur lesquels on peut aisément fixer de la lysine.

On a utilisé le produit connu sous la dénomination commerciale FICOLL et on a fixé tout d'abord de la lysine sur ce produit en faisant réagir des quantités en poids sensiblement égales de lysine et de FICOLL selon la technique décrite par SANDERSON C.I. et WILSON D.V.—A simple method for coupling protein to insoluble polysaccharides-Immunology 1971 Vol. 20 p.1061—1065.; on a ensuite traité le produit résultant selon le mode opératoire décrit à l'exemple 1 afin de fixer les groupes phosphorylcholine sur ce produit.

On a procédé selon le mode opératoire décrit ci-dessus en utilisant comme produit de départ le dextrane de poids moléculaire 500.000 environ.

Les composés de couplage obtenus ci-dessus ont donné des résultats sérologiques positifs par immunoprécipitations en gélose.

## Exemple 5

Protection des rats à la suite d'une prophylaxie médicamenteuse à l'aide des produits de couplage de l'invention.

Des rats ont reçu, par intubation stomacale à l'aide d'une canule possédant un bout en olive, 0,5 ml d'une solution physiologique contenant un produit de couplage de l'invention. Huit jours plus tard, ils ont été infestés, par voie sous cutanée, par 3000 larves du stade infestant $L_3$ du parasite *Nippostrongylus brasiliensis*. Ce parasite s'est installé, à l'état adulte, dans l'intestin grêle du rat et y a persisté jusqu'au 10ème jour après l'infestation.

Les manifestations classiques de l'immunité sont:
— l'arrêt de la ponte des parasites femelles;
— l'expulsion prématurée des adultes de l'intestion des rats.

On a donc procédé, afin d'estimer la protection conféré par les produits de l'invention, aux deux tests suivants:
— comptage journalier des oeufs émis dans le fèces d'une cage de rats ayant subi le même traitement.
— comptage de la population des vers de l'intestin de chaque rat 8 jours après l'infestation.

Les résultats du comptage de la population de vers adultes dans l'intestin des rats ont ensuite été soumis à un calcul statistique pour comparer l'efficacité des différents traitements.

Les tableaux suivants résument les résultats obtenus avec PC HSA$_0$.
Les rats du groupe I ont reçu 100 $\mu$g de PC HSA$_0$
Les rats du groupe II ont reçu 50$\mu$g de PC HSA$_0$
Les rats du groupe III ont reçu 10 $\mu$g de PC HSA$_0$
Les rats des groupes IV, V, VI, VII sont des témoins ayant reçu de la sérumalbumine ou du tampon physiologique.

On a indiqué dans le tableau I ci-après le nombre d'oeufs par gramme de fèces; les résultats de ce tableau I montrent que les animaux du groupe II, qui ont reçu 50 $\mu$g de PC HSA$_0$, ont excrété beaucoup moins d'oeufs que les autres.

7

## 0 001 197

Dans le tableau II, on a indiqué le nombre de vers à l'autopsie au jour 8 après l'infestation d'épreuve; ces résultats montrent que les animaux du groupe II ont hébergé moins de parasites que les autres.

L'ensemble de ces résultats montre que 50 $\mu$g de PC HSA$_Q$ protègent les animaux alors que 10 ou 100 $\mu$g ne les protègent pas.

TABLEAU I

Récolte des oeufs par gramme de fèces

| Groupe | jours après l'infestation | | |
|--------|--------|--------|--------|
| | 6 | 7 | 8 |
| I | 31200 | 120600 | 159800 |
| II | 3850 | 1170 | 23000 |
| III | 36950 | 112500 | 114600 |
| TEMOINS | | | |
| IV | 40750 | 94000 | 103000 |
| V | 44200 | 70400 | 102400 |
| VI | 45950 | 120500 | |
| VII | 51300 | 67900 | 108400 |

TABLEAU II

Nombre de vers à l'autopsie au jour 8 après l'infestation d'épreuve (moyenne)

| | |
|--------|--------|
| groupe I | 1125 |
| groupe II | 206 |
| groupe III | 942 |
| groupe IV | 874 |
| groupe V | 1036 |
| groupe VI | 1028 |
| groupe VII | 1531 |

8

Dans un autre type d'expérience, les cinque composés PC HSA$_1$ à PC HSA$_5$ ont été intubés à des groupes de rats à raison de 10 $\mu$g/rat huit jours avant une infestation d'épreuve de 2000 larves infestantes.

| groupe d'animaux traités | produit de couplage utilisé |
|---|---|
| groupe I | PC HSA$_1$ |
| groupe II | PC HSA$_2$ |
| groupe III | PC HSA$_3$ |
| groupe IV | PC HSA$_4$ |
| groupe V | PC HSA$_5$ |
| groupe VI | témoins |

Dans les tableaux III et IV ci-après, on a indiqué respectivement le nombre d'oeufs excrétés par gramme de fèces et le nombre de vers à l'autopsie au jour 8 après l'infestation.

Ces résultats montrent que les animaux ayant reçu 10 $\mu$g du composé PC HSA$_5$ ont excrété moins d'oeufs et ont hébergé moins de parasites que les autres animaux; le produit de couplage PC HSA$_5$ confère donc à la dose de 10 $\mu$g/rat la meilleure protection.

Ces essais montrent que les doses à utiliser varient en fonction du degré de substitution de la phosphorylcholine sur la sérum albumine humaine.

Des essais réailisés avec les produits de couplage hémocyanine-cytidine-diphosphocholine et gélatine-cytidine-diphosphocholine préparés selon les exemples 2 et 3 ont montré qu'ils étaient actifs en prophylaxie médicamenteuse.

TABLEAU III

Excrétion des oeufs par gramme de fèces récolté

| groupe | jours après l'infestation | |
|---|---|---|
| | 7 | 8 |
| I | 56000 | 48800 |
| II | 107600 | 86800 |
| III | 40400 | 86800 |
| IV | 17800 | 40400 |
| V | 4200 | 17800 |
| VI | 75800 | 64400 |

**0 001 197**

TABLEAU IV

Nombre de vers à l'autopsie au jour 8 après l'épreuve

| groupe | nombre de vers |
|---|---|
| groupe I | 757 |
| groupe II | 1043 |
| groupe III | 1233 |
| groupe IV | 560 |
| groupe V | 318 |
| groupe VI | 1003 |

Exemple 6

On a appliqué le procédé général décrit à l'exemple 1 pour coupler la cytidine-diphosphocholine et l'ovalbumine. On a ainsi obtenu des produits de couplage PC-ovalbumine, utilisables d'une manière analogue à celles de l'exemple 3 ou de l'exemple 5.

Exemple 7

On a appliqué le mode opératoire décrit à l'exemple 1 pour coupler la cytidine-diphosphocholine et l'inhibiteur de la trypsine du soja. Le produit de couplage obtenu trouve des applications du genre décrit précédemment à l'exemple 3 ou 5.

Exemple 8

On a appliqué le mode opératoire décrit à l'exemple 1 pour coupler la cytidine-diphosphocholine et le bacille tuberculeux tué.

Ce produit de couplage trouve des applications du genre décrit précédemment à l'exemple 3 ou 5.

Exemple 9

On a appliqué le mode opératoire décrit à l'exemple 1 pour couper la cytidine-diphosphocholine et *Bordetella pertussis* tué.

Ce produit de couplage trouve des applications du gentre décrit précédemment à l'exemple 3 ou 5.

Les produits de couplage selon l'invention, notamment ceux décrits dans les exemples précédents, sont capables d'induire chez les animaux une synthèse d'anticorps anti-phosphorylcholine.

Exemple 10

La cytidine-diphosphocholine activée par le périodate de sodium entraîne une sensibilité de contact de type retardé lorsqu'elle est administrée à l'animal par peinture cutanée.

Cette observation implique l'induction d'une immunité de type cellulaire.

Exemple 11

On a appliqué le mode opératoire décrit à l'exemple 1 pour couper la cytidine-diphosphocholine ou du Sépharose AH$_2$. Ce produit peut être utilisé en colonne pour purifier de la protéine C-réactive ou des anticorps anti-phosphorylcholine en une étape à partir des sérums les contenant. La protéine C réactive liée par le composé est relarguée par du citrate trisodique 5%, puis les anticorps anti-phosphorylcholine sont élués par de la phosphorylcholine libre $10^{-2}$ M.

**Revendications**

1. Produits de couplage de cytidine-diphosphocholine et de composés aminés portant des résidus lysine, la liaison de couplage se faisant, après oxydation de la cytidine-diphosphocholine, entre les groupes ribose oxydés de cette dernière et les résidus lysine desdits composés aminés.

2. Produits selon la revendication 1, caractérisés en ce que les composés portant des résidus lysine sont des composés obtenus par Fixetion de résidus lysine sur des composés sur lesquels on peut fixer des résidus lysine.

3. Produits selon la revendication 1, caractérisés en ce que les composés aminés portant des résidus lysine sont choisis parmi les albumines des différentes espèces animales, notamment la sérum albumine humaine, l'ovalbumine, la gélatine, les lectines comme la concanavaline A, l'hémocyanine, la limule, les corps bactériens, les flagelles de salmonelles, les bactériophages, les virus, les enzymes, l'inhibiteur de la trypsine, en particulier du soja, les polymères de la lysine ayant un degré de polymérisation supérieur à 2, les polymères synthétiques d'acides aminés possédant plusieurs résidus de lysine.

4. Produits selon la revendication 2, caractérisés en ce que les composés sur lesquels on peut fixer de la lysine sont choisis parmi des polysaccharides, tels que les dextranes, les levanes, les polymères de sucrose et d'épichlorydrine.

5. Produits de couplage selon l'une quelconque des revendications 1 à 4, caractérisés en ce qu'ils sont obtenus par le procédé qui consiste:

1) à oxyder de la cytidine-diphosphocholine;

2) à fixer le dialdéhyde, obtenu par l'oxydation précédente du ribose de la cytidine, sur les groupements $NH_2$ du composé aminé en solution aqueuse alcaline;

3) à stabiliser le couplage ainsi réalisé par réduction;

4) à récupérer le produit macromoléculaire ainsi obtenu sous forme d'une solution aqueuse, celle-ci pouvant être lyophilisée pour fournir une poudre apte à la conservation.

6. Produits de couplage selon la revendication 5, caractérisés en ce que l'oxydation est réalisée à l'aide de periodate de sodium et en ce que la stabilisation du couplage a lieu en présence d'un agent réducteur, tel que le borohydrure de sodium.

7. Produits de couplage selon l'une quelconque des revendications 1, 3 et 5, caractérisés en ce que le composé aminé est de la sérum albumine humaine, en ce que le rapport des restes de phosphorylcholine au nombre de moles de sérum albumine humaine est compris entre 1 et 30, lesdits produits se présentant sous la forme d'une poudre.

8. Procédé pour l'obtention des produits de couplage selon l'une quelconque des revendications 1 à 7, caractérisé en ce qu'il consiste à coupler de la cytidine-diphosphocholine, après oxydation, avec des composés aminés portant des résidus lysine, le couplage se faisant entre les groupes ribose oxydés de la cytidine-diphosphocholine et les résidus lysine desdits composés aminés.

9. Procédé selon la revendication 8, caractérisé en ce qu'il consiste:

1) à oxyder dela cytidine-diphosphocholine;

2) à fixer le dialdéhyde, obtenu par l'oxydation précédente du ribose de la cytidine, sur les groupements $NH_2$ du composé aminé en solution aqueuse alcaline;

3) à stabiliser le couplage ainsi réalisé par réduction,

4) à récupérer le produit macromoléculaire ainsi obtenu sous forme d'une solution aqueuse, celle-ci pouvant être lyophilisée pour fournir une poudre apte à la conservation.

10. Procédé selon l'une des revendications 8 ou 9, caractérisé en ce que l'oxydation est réalisée à l'aide de periodate de sodium et en ce que la stabilisation du couplage a lieu en présence d'un agent réducteur, tel que le borohydrure de sodium.

11. Application des produits de couplage selon l'une quelconque des revendications 1 à 7 au dosage de composés biologiques réagissant avec la phosphorylcholine.

12. Réactif de dosage de composés biologiques réagissant avec la phosphorylcholine, caractérisé en ce qu'il comprend un produit de couplage selon l'une quelconque des revendications 1 à 7, fixé sur des globules rouges de mouton éventuellement traités par le glutaraldéhyde.

13. Réactif selon la revendication 12 caractérisé en ce que le produit de couplage est activé par le glutaraldéhyde avant fixation sur des globules rouges de mouton éventuellement traités par du glutaraldéhyde.

14. Réactif de dosage de composés biologiques réagissant avec de la phosphorylcholine, caractérisé en ce qu'il comprend une plaque de gélose contenant un produit de couplage phosphorylcholine-produit aminé selon l'une quelconque des revendications 1 à 7.

15. Vaccin antiparasitaire pour protéger contre les parasites appartenant à ceux comportant des molécules liées à un residu phosphorylcholine, caractérisé en ce qu'il contient une quantité efficace d'un produit de couplage selon l'une quelconque des revendications 1 à 7, en combinaison avec un véhicule pharmaceutiquement acceptable.

16. Vaccin selon la revendication 15, caractérisé en ce que le véhicule est du sérum physiologique ou un tampon aqueux, ou un produit d'enrobage présenté sous forme de gélules ou de liposomes.

**Patentansprüche**

1. Kupplungsprodukte aus Cytidin-Diphosphochilin und aminierten Verbindungen, die Lysinreste enthalten, wobei die Kupplungsbildung nach der Oxidation des Cytidin-Diphosphocholins zwischen den oxidierten Ribosegruppen des letzteren und den Lysinresten der aminierten Verbindungen stattfindet.

2. Kupplungsprodukte nach Anspruch 1, dadurch gekennzeichnet, daß die Verbindungen mit Lysinresten solche sind, die durch Fixierung der Lysinreste auf Verbindungen erhalten wurden, auf denen Lysinreste fixiert werden können.

3. Kupplungsprodukte nach Anspruche 1, dadurch gekennzeichnet, daß die aminierten, Lysinreste tragenden Verbindungen ausgewählt sind aus Albuminen unterschiedlicher Tierarten, insbesondere menschliches Serumalbumin, Ovalbumin, Gelatine, Lectinen, wie Concanavalin A, Hämocyanin, Limulus poly phemus Bakterienkörper, Salmonellenflagellen, Bakteriophagen, Viren, Enzyme, Trypsininhibitor, insbesondere Soja, Lysinpolymere mit einem Polymerisationsgrad über 2, synthetische Polymere von Aminosäuren mit mehreren Lysinresten.

4. Kupplungsprodukte nach Anspruch 2, dadurch gekennzeichnet, daß die Verbindungen, auf denen man Lysin fixieren kann, ausgewählt sind aus Polysacchariden, wie Dextrane, Lävane, Polymere von Saccharose und Epichlorhydrin.

5. Kupplungsprodukte nach einem der Anspruche 1 bis 4, dadurch gekennzeichnet, daß sie nach einem Verfahren erhalten worden sind, bei welchem man
1) das Cytidin-Diphosphocholin oxidiert;
2) den durch vorhergehende Oxidation von Cytidinribose erhaltenen Dialdehyd in wässriger alkalischer Lösung auf den $NH_2$ Gruppen der aminierten Verbindung fixiert;
3) die so erhaltene Kupplung durch Reduktion stabilisiert und
4) das so erhaltene makromolekulare Produkt in Form einer wässrigen Lösung gewinnt, die zur Bildung eines zur Konservierung geeigneten Pulvers lyophilisiert werden kann.

6. Kupplungsprodukte nach Anspruch 5, dadurch gekennzeichnet, daß die Oxidation mittels Natriumperjodat erfolgt und die Stabilisierung der Kupplung in Anwesenheit eines Reduktionsmittels, wie Natriumborhydrid, durchgeführt wird.

7. Kupplungsprodukte nach einem der Ansprüch 1, 3 und 5, dadurch gekennzeichnet, daß die Aminoverbindung menschliches Serumalbumin ist, daß das Verhältnis der Phosphorylcholinreste zur Molanzahl des menschlichen Serumalbumins zwischen 1 und 30 liegt und die Produkte in Form eines Pulvers vorliegen.

8. Verfahren zur Herstellung der Kupplungsprodukte nach einem der Ansprüch 1 bis 7, dadurch gekennzeichnet, daß man das Cytidin-Diphosphocholin nach Oxidation mit den aminierten, Lysinreste tragenden Verbindungen kuppelt, wobei die Kupplung zwischen den oxidierten Ribosegruppen des Cytidin-Diphosphochilins und den Lysinresten dieser aminierten Verbindungen erfolgt.

9. Verfahren nach Anspruch 8, dadurch gekennzeichnet, daß man
1) das Cytidin-Diphosphocholin oxidiert;
2) den durch vorhergehende Oxidation der Ribose des Cytidins erhaltenen Dialdehyd in wässriger alkalischer Lösung auf den $NH_2$ Gruppen der aminierten Verbindung fixiert;
3) die so erhaltene Kupplung durch Reduktion stabilisiert und
4) das so erhaltene makromolekulare Produkt in Form einer wässrigen Lösung gewinnt, die zur Bildung eines zur Konservierung geeigneten Pulvers lyophilisiert werden kann.

10. Verfahren nach einem der Ansprüche 8 und 9, dadurch gekennzeichnet, daß die Oxidation mit Hilfe von Natriumperjodat erfolgt und die Stabilisierung der Kupplung in Anwesenheit eines Reduktionsmittels, wie Natriumborhydrid, stattfindet.

11. Anwendung der Kupplungsprodukte nach einem der Ansprüche 1 bis 7 zum Bestimmen biologischer, mit dem Phosphorylcholin reagierender Verbindungen.

12. Bestimmungsmittel für biologische, mit dem Phosphorylcholin reagierende Verbindungen, dadurch gekennzeichnet, daß es ein Kupplungsprodukt nach einem der Ansprüche 1 bis 7 enthält, das auf roten Blutkörperchen von Hammeln, die gegebenenfalls mit Glutaraldehyd behandelt sind, fixiert ist.

13. Mittel gemäß Anspruch 12, dadurch gekennzeichnet, daß das Kupplungsprodukt vor der Fixierung auf den roten Blutkörperchen von Hammeln, die gegebenenfalls mit Glutaraldehyd behandelt worden sind, mit Glutaraldehyd aktiviert wird.

14. Bestimmungsmittel für biologische, mit Phosphorylcholin reagierende Verbindungen, dadurch gekennzeichnet, daß sie eine Agar-Platte umfaßt, welche ein aminiertes Phosphorylcholin Kupplungsprodukt nach einem der Ansprüche 1 bis 7 enthält.

15. Antiparasitäre Vaccine zum Schutz gegen Parasiten, die zu denjenigen gehören welche Moleküle enthalten, die an einen Phosphorylcholinrest gebunden sind, dadurch gekennzeichnet, daß es ein wirksame Menge eines Kupplungsproduktes nach einem der Ansprüche 1 bis 7 in Kombination mit einem pharmazeutisch annehmbaren Träger enthält.

16. Vaccine nach Anspruch 15, dadurch gekennzeichnet, daß der Träger ein physiologisches Serum oder ein wässriger Puffer oder ein umhülltes Produkt in Form Gelatinekapseln oder Liposomen ist.

**Claims**

1. Coupling products of cytidene-diphosphocholine and amino compounds bearing lysine

**0 001 197**

residues, the coupling bond occurring, subsequently to oxidation of the cytidine-diphosphocholine, between the oxidized ribose groups of the latter and the lysine residues of said amino compounds.

2. Products according to claim 1, characterized in that the compounds bearing lysine residues are compounds obtained by binding lysine residues to compounds whereto lysine residues may be bonded.

3. Products according to claim 1, characterized in that said amino compounds bearing lysine residues are selected among the albumins of the various animal species, especially human serum albumin, ovalbumin, gelatin, lectines such as concanavaline A, hemocyanine, Limulus polyphemus, bacterial bodies salmonellae flagellae, bacteriophages, viruses, enzymes, the trypsine inhibitor, in particular of soja, lysine polymers having a polymerization degree higher than 2, synthetic polymers of amino-acids having several lysine residues.

4. Products according to claim 2, characterized in that said compounds whereto lysine may be bonded are selected among polysaccharides such as dextranes, levanes, sucrose polymers, and epichlorohydrin polymers.

5. Coupling products according to any one of claims 1 to 4, characterized in that they are obtained by the process consisting in:

1) oxydizing the cytidine-diphosphocholine;

2) binding the dialdehyde, obtained by the previous oxidation of the ribose in the cytidine, to the $NH_2$ groups of the amino compound in an alkaline aqueous solution;

3) Stabilizing the thus effected coupling by reduction;

4) recovering the macromolecular thus obtained product under the form of an aqueous solution, the latter being apt to be lyophilized for providing a preservable powder.

6. Coupling products according to claim 5, characterized in that the oxidation is effected by means of sodium periodate and in that the coupling stabilization occurs in the presence of a reducing agent, such as sodium borohydride.

7. Coupling products according to anyone of claims 1, 3 and 5, characterized in that said amino compound is human serum albumin, in that the ratio of the residues of phosphorylcholine to the number of moles of human serum alubmin ranges from 1 to 30, said products being under the form of a powder.

8. A method for the obtention of coupling products according to anyone of claims 1 to 7, characterized in that it consists in coupling the citidine-diphosphocholine, subsequently to oxidation, with amino compounds bearing lysine residues, the coupling occurring between the oxidized ribose groups of the cytidine-diphosphocholine and the lysine residues in said amino compounds.

9. Method according to claim 8, characterized in that it consists in:

1) oxidizing the cytidine-diphosphocholine,

2) binding the dialdehyde, obtained by the previous oxidation of the ribose in the cytidine, to the $NH_2$ groupings of the amino compound in an alkaline aqueous solution;

3) stabilizing the thus effected coupling by reduction;

4) recovering the thus obtained macromolecular product under the form of an aqueous solution, the latter being apt to be lyophilized to provide a preservable powder.

10. Method according to anyone of claims 8 or 9, characterized in that the oxidation is effected by means of sodium periodate and in that the coupling stabilization occurs in the presence of a reducing agent, such as sodium borohydride.

11. Use of the coupling products according to anyone of claims 1 to 7 to the assay of biological compounds which react with phosphorylcholine.

12. Reagent for the assay of biological compounds which react with phosphorylcholine, characterized in that it comprises a coupling product as claimed in anyone of claims 1 to 7, bonded to sheep's red blood cells optionally treated with glutaraldehyde.

13. Reagent according to claim 12, characterized in that the coupling product is activated with glutaraldehyde before being bonded to sheep's red blood cells optionally treated with glutaraldehyde.

14. Reagent for the assay of biological compounds which react with phosphorylcholine, characterized in that it comprises a plate of agar-agar containing a coupling product of phosphorylcholine-amino compound according to anyone of claims 1 to 7.

15. An antiparasitic vaccine for protection against parasites of the group including molecules bonded to the a phosphorylcholine residue, characterized in that it contains an effective amount of a coupling product according to anyone of claims 1 to 7, in combination with a pharmaceutically acceptable carrier.

16. A vaccine according to claim 15, characterized in that the carrier is a physiological salt solution or an aqueous buffer, or a coating product in the form of capsules or liposomes.

13